# NEUE EUROPÄISCHE PATENTSCHRIFT

(11) **EP 1 220 940 B2**
(45) Veröffentlichungstag und Bekanntmachung des Hinweises auf die Entscheidung über den Einspruch: **28.07.2010**
(45) Hinweis auf die Patenterteilung: 29.01.2003
(21) Anmeldenummer: 00969413.4
(22) Anmeldetag: 05.10.2000
(51) Int. Cl.: C12P 13/12, C12N 9/10, C12N 15/54, C12N 15/67, C12N 15/70

(54) **VERFAHREN ZUR FERMENTATIVEN HERSTELLUNG VON L-CYSTEIN ODER L-CYSTEIN-DERIVATEN**
METHOD FOR PRODUCTION OF L-CYSTEINE OR L-CYSTEINE DERIVATIVES BY FERMENTATION
PROCEDES POUR PRODUIRE DE LA L-CYSTEINE OU DES DERIVES DE CETTE DERNIERE PAR FERMENTATION

(30) Priorität: 14.10.1999 DE 19949579
(43) Veröffentlichungstag der Anmeldung: 10.07.2002
(73) Patentinhaber: Wacker Chemie AG, 81737 München (DE)
(72) Erfinder: MAIER, Thomas, 85221 Dachau (DE); WINTERHALTER, Christoph, 82343 Pöcking (DE)
(74) Vertreter: Potten, Holger
(86) Internationale Anmeldenummer: PCT/EP2000/009720
(87) Internationale Veröffentlichungsnummer: WO 2001/027307

(56) Entgegenhaltungen:
- EP-A- 0 885 962
- WO-A-97/15673
- TOPCZEWSKI ET AL: "Cloning and characterization of the Aspergillus nidulans cysB gene encoding cysteine synthase" CURRENT GENETICS,US,NEW YORK, NY, Bd. 31, Nr. 4, April 1997 (1997-04), Seiten 348-356, XP002109934 ISSN: 0172-8083
- NAKAMORI ET AL: "overproduction of L-cysteine and L-cystine by Escherichia coli strains with a genetically altered serine acetyltransferase" APPLIED AND ENVIRONMENTAL MICROBIOLOGY,US,WASHINGTON,DC, Bd. 64, Nr. 5, Mai 1998 (1998-05), Seiten 1607-1611, XP002115630 ISSN: 0099-2240 in der Anmeldung erwähnt
- DENK D ET AL: "L-CYSTEINE BIOSYNTHESIS IN ESCHERICHIA COLI: NUCLEOTIDE SEQUENCE AND EXPRESSION OF THE SERINE ACETYLTRANSFERASE (CYSE) GENE FROM THE WILD-TYPE AND A CYSTEINE-EXCRETING MUTANT" JOURNAL OF GENERAL MICROBIOLOGY,GB,SOCIETY FOR MICROBIOLOGY, READING, Bd. 133, Nr. 3, 1. März 1987 (1987-03-01), Seiten 515-525, XP000617605 ISSN: 0022-1287
- SIRKO A E ET AL: "IDENTIFICATION OF THE ESCHERICHIA COLI CYSM GENE ENCODING O-ACETYLSERINE SULPHYDRYLASE B BY CLONING WITH MINI-MU-LAC CONTAINING A PLASMID REPLICON" JOURNAL OF GENERAL MICROBIOLOGY,GB,SOCIETY FOR MICROBIOLOGY, READING, Bd. 133, Nr. 10, 1. Oktober 1987 (1987-10-01), Seiten 2719-2725, XP000618706 ISSN: 0022-1287
- SCHELL MARK A: "Molecular biology of the LysR family of transcriptional regulators." ANNUAL REVIEW OF MICROBIOLOGY, Bd. 47, 1993, Seiten 597-626, XP000992127 1993 Annual Reviews Inc. P.O. Box 10139, 4139 El Camino Way, Palo Alto, California 94306, USA ISBN: 0-8243-1147-7 in der Anmeldung erwähnt
- DASSLER TOBIAS ET AL: "Identification of a major facilitator protein from Escherichia coli involved in efflux of metabolites of the cysteine pathway." MOLECULAR MICROBIOLOGY, Bd. 36, Nr. 5, Juni 2000 (2000-06), Seiten 1101-1112, XP000992212 ISSN: 0950-382X

### SEQUENZPROTOKOLL

<110> Consortium fuer elektrochemische Industrie GmbH
<120> Verfahren zur fermentativen Herstellung von L-CyStein oder L-Cystein-Derivaten
<130> Co9905
<140>
   <141>
<160> 5
<170> PatentIn Vers. 2.0
<210> 1
   <211> 18
   <212> DNA
   <213> Artificial Sequence
<220>
   <223> Primer for PCR
<400> 1
   gttacgagat cgaagagg 18
<210> 2
   <211> 18
   <212> DNA
   <213> Artificial Sequence
<220>
   <223> Primer for PCR
<400> 2
   gtcaccgagt ggtcaatg 18
<210> 3
   <211> 27
   <212> DNA
   <213> Artificial Sequence
<220>
   <223> oligonueleotide for in vitro mutagenensis
<400> 3
   ttcgctatcg ccatggaagc getgcat 27
<210> 4
   <211> 27
   <212> DNA
   <213> Artificial Sequence
<220>
   <223> Primer for PCR
<400> 4
   ccggaattcc cgttgccgtt tgtggcg 27
<210> 5
   <211> 27
   <212> DNA
   <213> Artificial Sequence
<220>
   <223> Primer for PCR
<400> 5
   cgcggatccg tgtgaccgat agtcagc 27

## Beschreibung

Die Erfindung betrifft Verfahren zur Herstellung von L-Cystein oder L-Cystein-Derivaten mittels Fermentation von Mikroorganismen sowie für das Verfahren geeignete Mikroorganismen.

Die Aminosäure L-Cystein ist von wirtschaftlicher Bedeutung. Sie wird beispielsweise als Lebensmittelzusatzstoff (insbesondere in der Backmittelindustrie), als Einsatzstoff in der Kosmetik, sowie als Ausgangsprodukt für die Herstellung von Pharmawirkstoffen (insbesondere N-Acetyl-Cystein und S-Carboxy-Methyl-Cystein) verwendet.

L-Cystein-Derivate sind alle S-haltigen Metaboliten die sich in ihrer Synthese vom Cystein ableiten, also z.B. Cystin, Methionin, Glutathion, Biotin, Thiazolidine, Thiamin, Liponsäure und Coenzym A.

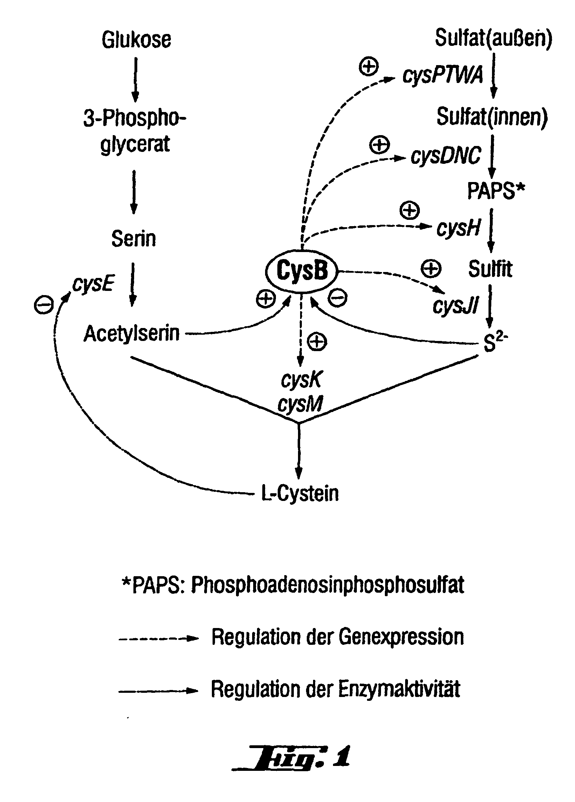

Die Regulation der Cysteinbiosynthese in Bakterien erfolgt auf zwei Ebenen (Fig. 1) :
1. Auf der Ebene der Enzymaktivität unterliegt die Serin-Acetyl-Transferase (cysE-Genprodukt) einer Endprodukt-Hemmung durch L-Cystein. Dies bedeutet, daß eine Akkumulation von L-Cystein direkt zur Hemmung der ersten spezifischen Reaktion der Cystein-Biosynthese führt und die weitere Synthese unterbunden wird.
2. Auf der Ebene der Transkription fungiert das Regulatorprotein CysB (kodiert durch das cysB-Gen) als Transkriptionsaktivator und sorgt für eine regulierte Bereitstellung von reduziertem Schwefel. Als Induktor benötigt CysB N-Acetyl-Serin, das in der Zelle aus O-Acetyl-Serin entsteht, wenn nicht ausreichend reduzierter Schwefel für die O-Acetyl-Serin-Sulfhydrylase-Reaktion zur Verfügung steht. Folglich stehen alle Gene, die mit Aufnahme, Reduktion und Einbau von Schwefel in Verbindung stehen, unter der Kontrolle von CysB. Dies sind die Operone: cysPTWAM, cysDNC, cysJIH und das cysK-Gen. Während Acetyl-Serin als Induktor für CysB wirkt, zeigen Sulfid und Thiosulfat einen negativen Effekt als sogenannte "Antiinducer", da ihr Vorhandensein die Verfügbarkeit von SH-Gruppen anzeigt.

Der Stand der Technik bezüglich der Gewinnung von L-Cystein und L-Cystein-Derivaten wird ausführlich in WO 97/15673 (entspricht der US Anmeldung SN 09/065104) diskutiert. WO 97/15673 selbst beschreibt ein fermentatives Herstellungsverfahren, bei dem feedbackresistente Serin-Acetyl-Transferasen zum Einsatz kommen. Die Anmeldung offenbart ferner, daß eine weitere Steigerung der Cysteinausbeute durch eine zusätzliche Deregulierung des Regulatorproteins CysB auf Genebene im Sinne einer konstitutiven Expression möglich ist.

Die Patentanmeldung EP 885962 A1 (entspricht der US Anmeldung mit der Serial number SN 09/097759) offenbart Mikroorganismen, die zur fermentativen Herstellung von L-Cystein, L-Cystin, N-Acetyl-Serin und Thiazolidinderivaten geeignet sind, die dadurch gekennzeichnet sind, daß sie mindestens ein Gen überexprimieren, das für ein Protein kodiert, welches direkt zur Ausschleusung von Antibiotika oder anderen für die Mikroorganismen toxischen Stoffen aus der Zelle geeignet ist.

CysB gehört zur Familie der LysR-Typ-Transkriptionsregulatoren (LTTR) von der bereits über 100 Vertreter bekannt sind (Schell M. A., 1993, Annu. Rev. Microbiol. 47: 597-626). Sie zeichnen sich durch eine N-terminale DNS-Bindedomäne mit einem Helix-Turn-Helix-Motiv und eine C-terminale Induktor-Bindungsdomäne aus. Von CysB liegen detaillierte DNS-Bindungsstudien vor. Zudem ist CysB das erste LTTR-Protein, von dem auch eine Kristallstruktur zur Verfügung steht (Tyrell et al., 1997, Structure 5:1017-1032). LTTR-Proteine wirken in der Regel als positive Genregulatoren, die vom Vorhandensein eines Induktormoleküls abhängig sind. Es wurde bisher angenommen, daß nur hochaktive CysB-Varianten, die unabhängig von Effektormolekülen sind (sogenannte konstitutiv aktive Varianten), eine Steigerung der Cystein-Produktion zulassen, da solche Formen gleichbleibend hohe Genaktivierung entfalten (Nakamori S. et al., 1998, Appl. Env. Microbiol. 64:1607-1611).

Beispiele für konstitutiv aktive Formen von CysB wurden für Salmonella typhimurium beschrieben. Diese Varianten zeigen hohe Aktivität, wobei die Aktivität völlig unabhängig vom Induktor N-Acetyl-Serin und der negativen Wirkung von Thiosulfat und Sulfid ist (Colyer T. E., Kredich N. M., 1994, Mol. Microbiol. 13: 797-805).

Die vorliegende Erfindung betrifft einen Mikroorganismenstamm, der zur fermentativen Herstellung von L-Cystein oder L-Cystein-Derivaten geeignet ist und einen deregulierten Cysteinstoffwechsel besitzt, wobei diese Deregulation des Cysteinstoffwechsels nicht auf einer geänderten CysB-Aktivität beruht, dadurch gekennzeichnet, daß er zusätzlich eine erhöhte CysB-Aktivität besitzt, wobei die CysB-Aktivität ein für ein Wildtyp-CysB typisches Regulationsmuster besitzt.

Mikroorganismenstämme mit dereguliertem Cysteinstoffwechsel bei denen diese Deregulation nicht auf einer veränderten CysB-Aktivität beruht sind bekannt. Es handelt sich um Stämme mit modifizierten cysE-Allelen wie beispielsweise in WO 97/15673 oder Nakamori S. et al., 1998, Appl. Env. Microbiol. 64: 1607-1611 beschrieben, oder um Stämme, bei denen Efflux-Gene eingesetzt werden wie beispielsweise in EP 0885962 A1 (entspricht der US Anmeldung mit der Serial Number SN 09/097759 beschreiben, oder um Stämme, die unter Einsatz unspezifischer Mutagenese-Methoden kombiniert mit Screening-Methoden für Cystein-Überproduktion oder verminderten Cystein-Abbau gewonnen werden, wie beispielsweise in WO 97/15673 oder in Nakamori S. et al., 1998, Appl. Env. Microbiol. 64:1607-1611 beschrieben.

Im Sinne der Erfindung ist eine erhöhte CysB-Aktivität gegeben, wenn die Aktivität von CysB um mindestens 10 % im Vergleich zur CysB-Aktivität im Wildtypstamm erhöht ist.

Bevorzugt ist die CysB-Aktivität um mindestens 25 % erhöht.

Besonders bevorzugt ist die CysB-Aktivität um mindestens 50 % erhöht.

Ein Escherichia coli Stamm (MC4100::λKZL300) der zur Bestimmung der CysB-Aktivität geeignet ist, wird in Beispiel 2 beschrieben. Er wurde bei der DSMZ (Deutsche Sammlung für Mikroorganismen und Zellkulturen GmbH, D-38142 Braunschweig) gemäß Budapester Vertrag am 23.6.99 unter der Nummer DSM 12886 hinterlegt. Das jeweilige cysB-haltige Konstrukt wird dazu in den Stamm MC4100::λKZL300 in an sich bekannter Art und Weise eingebracht.

Ein für ein Wildtyp-CysB typisches Regulationsmuster der CysB-Aktivität ist in Abhängigkeit von der S-Quelle gegeben, wenn die CysB-Aktivität von Zellen gewachsen mit Thiosulfat gegenüber Zellen gewachsen mit Sulfat weniger als 75% beträgt und die CysB-Aktivität von Zellen gewachsen mit Cystin gegenüber Zellen gewachsen mit Sulfat weniger als 20% beträgt (siehe Beispiel 2).

Erfindungsgemäße Mikroorganismenstämme sezernieren L-Cystein oder ein L-Cystein Derivat im Vergleich zu einem Mikroorganismenstamm mit dereguliertem Cysteinstoffwechsel ohne erhöhte CysB-Aktivität in erhöhtem Ausmaß.

Ein erfindungsgemäßer Mikroorganismenstamm ist demnach ein Mikroorganismenstamm mit dereguliertem Cysteinstoffwechsel, wobei diese Deregulation des Cysteinstoffwechsels nicht auf einer veränderten CysB-Aktivität beruht, in dem homologe oder heterologe cysB-Gene verstärkt exprimiert werden, die für CysB mit einem Wildtyp-CysB typischen Regulationsmuster kodieren.

Bevorzugt handelt es sich um Escherichia coli Stämme mit dereguliertem Cysteinstoffwechsel, wobei diese Deregulation des Cysteinstoffwechsels nicht auf einer veränderten CysB-Aktivität beruht, in denen ein Wildtyp-cysB-Gen überexprimiert wird.

Besonders bevorzugt handelt es sich um Escherichia coli Stämme mit dereguliertem Cysteinstoffwechsel, wobei diese Deregulation des Cysteinstoffwechsels nicht auf einer veränderten CysB-Aktivität beruht, in denen die Kopienzahl des Wildtyp-cysB-Gens von Escherichia coli erhöht ist und dieses Gen überexprimiert wird.

Es zeigte sich überraschend, daß nicht wie in den genannten Offenbarungen des Stands der Technik postuliert, ein Einsatz von cysB-Allelen, die für konstitutiv aktive CysB-Regulatorproteine kodieren, die Cystein-Produktion steigert, sondern daß, ganz im Gegenteil, eine verstärkte Expression eines Wildtyp-cysB-Gens die Cystein-Produktion steigert.

Dieser Befund ist auch deshalb überraschend und unerwartet, da bisher kein Beispiel bekannt ist, in dem eine vermehrte Expression eines Regulatorproteins aus der Familie der LysR-Typ-Transkriptionsregulatoren eine Überproduktion eines Metaboliten ermöglicht.

Die Erfindung betrifft somit auch die Verwendung eines Regulatorproteins aus der Familie der LysR-Typ-Transkriptionsregulatoren zur Überproduktion eines Metaboliten sowie ein Verfahren zur Überproduktion eines Metaboliten, das dadurch gekennzeichnet ist, daß ein Regulatorgen aus der Familie der LysR-Typ-Transkriptionsregulatoren in einem Mikroorganismus überexprimiert wird und eine verstärkte Produktion des Metaboliten im Mikroorganismus bewirkt.

Eine Erhöhung der CysB-Aktivität in einem Mikroorganismus bei gleichzeitigem Erhalt des typischen Regulationsmusters kann zum Beispiel erreicht werden durch:
1. eine Erhöhung der Kopienzahl eines cysB-Gens, kodierend für CysB mit einem Wildtyp-CysB typischen Regulationsmuster, im Mikroorganismus unter Kontrolle eines Promotors oder
2. eine verstärkte Expression eines cysB-Gens kodierend für CysB mit einem Wildtyp-CysB typischen Regulationsmuster durch Austausch des die Expression des Wildtyp-cysB-Gens regulierenden Promotors gegen einen stärkeren Promotor.

cysB-Gene sind bekannt aus Escherichia coli, Salmonella typhimurium, Klebsiella aerogenes, Haemophilus influenzae, Pseudomonas aeruginosa und Thiocapsa roseopersicina. Als cysB-Gene werden vorzugsweise Gene verstanden, deren Genprodukte mit dem CysB-Protein von Escherichia coli eine Identität von mindestens 40 % aufweisen. Die Homologiewerte beziehen sich auf Ergebnisse, die mit dem Computerprogramm "Wisconsin Package Version 9.0, Genetics Computer Group (GCG), Madison, Wisconsin" erhalten werden. Dabei erfolgt die Suche in der Datenbank mit dem Unterprogramm "blast" unter Verwendung der Standardparameter.

cysB-Gene sind ferner solche Allele von Wildtyp-cysB-Genen, deren Genprodukte in ihrer Sequenz verändert sind, ohne daß dadurch das typische Wildtyp-CysB Regulationsmuster verloren geht. Ein Beispiel hierfür sind CysB-Varianten mit konservativen Aminosäure-Austauschen.

Ein erfindungsgemäßer Mikroorganismus läßt sich beispielsweise dadurch herstellen, daß in einem Mikroorganismenstamm mit dereguliertem Cysteinstoffwechsel, wobei diese Deregulation des Cysteinstoffwechsels nicht auf einer veränderten CysB-Aktivität beruht, mittels an sich bekannter Methoden die Kopienzahl des Wildtyp-cysB-Gens oder eines cysB-Gens kodierend für CysB mit einem Wildtyp-CysB typischen Regulationsmuster erhöht wird, oder daß mittels an sich bekannter Methoden eine verstärkte Expression des Wildtyp-cysB-Gens oder eines cysB-Gens kodierend für ein CysB mit einem Wildtyp-CysB typischen Regulationsmuster bewirkt wird.

Im folgenden bedeutet der Begriff "CysB" sowohl "Wildtyp-CysB" als auch "CysB-Variante mit einem für einen Wildtyp-CysB typischem Regulationsmuster". Der Begriff "cysB-Gen" bedeutet im folgenden "Wildtyp-cysB-Gen" sowie "cysB-Gen kodierend für CysB mit einem für Wildtyp-CysB typischen Regulationsmuster".

Die Erhöhung der Kopienzahl des cysB-Gens in einem Mikroorganismus kann mit dem Fachmann bekannten Methoden vorgenommen werden. So kann zum Beispiel das cysB-Gen in Plasmid-Vektoren mit mehrfacher Kopienzahl pro Zelle (z.B. pUC19, pBR322, pA-CYC184) kloniert und in einen Mikroorganismus mit dereguliertem Cysteinstoffwechsel eingebracht werden. Alternativ kann das cysB-Gen mehrfach ins Chromosom eines Mikroorganismus mit dereguliertem Cysteinstoffwechsel integriert werden. Als Integrationsverfahren können die bekannten Systeme mit temperenten Bakteriophagen, integrative Plasmide oder die Integration über homologe Rekombination genutzt werden (z.B. Hamilton et al., 1989, J. Bacteriol. 171: 4617-4622).

Bevorzugt ist die Erhöhung der Kopienzahl durch Klonierung eines cysB-Gens in Plasmid-Vektoren unter der Kontrolle eines Promotors. Besonders bevorzugt ist die Erhöhung der Kopienzahl durch Klonierung eines cysB-Gens in pACYC-Derivate wie z. B. pACYC184-LH (hinterlegt gemäß Budapester Vertrag bei der Deutschen Sammlung für Mikroorganismen und Zellkulturen, Braunschweig am 18.8.95 unter der Nummer DSM 10172).

Als Kontrollregion für die Expression eines plasmid-kodierten cysB-Gens kann die natürliche Promotor- und Operatorregion des Gens dienen.

Die verstärkte Expression eines cysB-Gens kann jedoch auch mittels anderer Promotoren erfolgen. Entsprechende Promotorsysteme sind dem Fachmann bekannt (Makrides S. C., 1996, Microbiol. Rev. 60: 512-538). Solche Konstrukte können in an sich bekannter Weise auf Plasmiden oder chromosomal verwendet werden.

Die Klonierung eines cysB-Gens in Plasmid-Vektoren erfolgt beispielsweise durch spezifische Amplifikation mittels der Polymerase-Ketten-Reaktion unter Einsatz von spezifischen Primmern, die das komplette cysB-Gen mit Promoter- und Operatorsequenz erfassen und anschließende Ligation mit Vektor-DNS-Fragmenten.

Als bevorzugte Vektoren für die Klonierung eines cysB-Gens werden Plasmide verwendet, die bereits genetische Elemente zur Deregulierung des Cysteinstoffwechsels enthalten, beispielsweise ein cysEX-Gen (WO97/15673) und ein Effluxgen (EP 0885962 Al). Solche Vektoren ermöglichen die Herstellung eines erfindungsgemäßen Mikroorganismenstammes aus einem beliebigen Mikroorganismenstamm, da ein solcher Vektor auch eine Deregulierung des Cysteinstoffwechsels in einem Mikroorganismus bewirkt.

Die Erfindung betrifft somit auch ein Plasmid, das dadurch gekennzeichnet ist, daß es genetische Elemente zur Deregulierung des Cysteinstoffwechsels besitzt, wobei diese genetischen Elemente keine Veränderung der CysB-Aktivität bewirken und nicht für eine Topoisomerase I kodieren, sowie ein cysB-Gen kodierend für CysB mit einem für Wildtyp CysB typischen Regulationsmuster unter Kontrolle eines Promotors enthält.

Durch eine gängige Transformationsmethode (z.B. Elektroporation) werden die cysB-haltigen Plasmide in Bakterien eingebracht und beispielsweise mittels Antibiotika-Resistenz auf plasmidtragende Klone selektiert.

Die Erfindung betrifft somit auch Verfahren zur Herstellung eines erfindungsgemäßen Mikroorganismenstammes, dadurch gekennzeichnet, daß in einen Mikroorganismenstamm ein erfindungsgemäßes Plasmid eingebracht wird.

Die Produktion von Cystein oder Cystein-Derivaten mit Hilfe eines erfindungsgemäßen Mikroorganismenstammes erfolgt in einem Fermenter nach an und für sich bekannten Verfahren. Als C-Quellen können z.B. Glukose, Laktose oder andere Zucker, als N-Quelle Ammonium oder Proteinhydrolysate verwendet werden. Als S-Quelle können z.B. Sulfid, Sulfit, Sulfat oder Thiosulfat verwendet werden. Während der Fermentation gebildetes L-Cystein kann durch Oxidation zu schwerlöslichem Cystin oder durch Kondensation mit Aldehyden oder Ketonen zu Thiazolidinen (z.B. mit Brenztraubensäure zu 2-Methyl-thiazolidin-2,4-dicarbonsäure) abreagieren.

Die Erfindung betrifft somit auch ein Verfahren zur Herstellung von L-Cystein, oder L-Cystein-Derivaten, welche dadurch gekennzeichnet sind, daß ein erfindungsgemäßer Mikroorganismenstamm in an sich bekannter Art und Weise in der Fermentation eingesetzt wird und das L-Cystein oder L-Cystein-Derivat aus dem Fermentationsansatz abgetrennt wird.

Die folgenden Beispiele dienen der weiteren Erläuterung der Erfindung.

### Beispiel 1: Klonierung des Wildtyp-cysB-Gens und des cysB(T149M)-Allels

Das Wildtyp-cysB-Gen von Escherichia coli wurde unter Anwendung der Polymerase-Ketten-Reaktion (PCR) kloniert. Mit den spezifischen Oligonukleotid-Primern (20 pmol pro Ansatz) cysBP1 (SEQ. ID.NO: 1) und cysBP2 (SEQ. ID.NO: 2) wurde ein genomisches 3107 Basenpaare langes DNS-Fragment amplifiziert, das das Wildtyp-cysB-Gen mit flankierenden Regionen umfaßt und terminale EcoRI- bzw. SalI-Restriktionsschnittstellen besitzt.

5´-GTT ACG AGA TCG AAG AGG-3´ (Phosphorothioat-Bindung am 3´-
Ende) (SEQ. ID.NO: 1)

5´-GTC ACC GAG TGG TCA ATG-3´ (Phosphorothioat-Bindung am 3´-
Ende) (SEQ. ID.NO: 2)

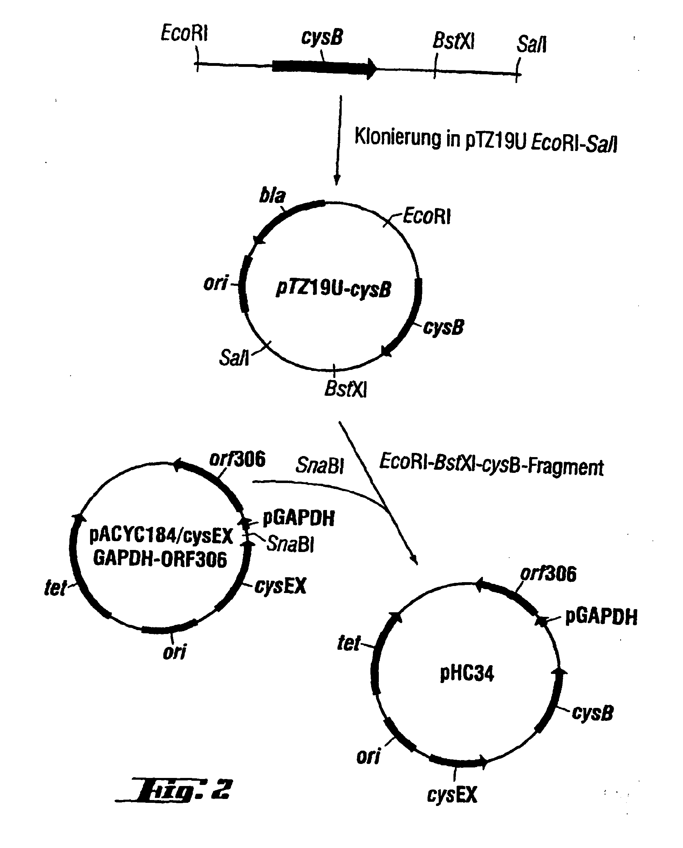

Die PCR-Reaktion wurde mit der Pwo-DNS-Polymerase der Firma Boehringer (Mannheim, D) mit 10 ng genomischer DNS als Matrize durchgeführt. Das Programm umfaßte 29 Zyklen mit einer Annealingtemperatur von 56 °C (30 Sekunden pro Zyklus), einer Extensionstemperatur von 72 °C (60 Sekunden pro Zyklus) und einer Denaturierungstemperatur von 94 °C (30 Sekunden pro Zyklus). Das DNS-Fragment wurde mit den Restriktionsenzymen EcoRI und SalI nachbehandelt und über eine präparative Gelelektrophorese und die Geneclean-Methode (Geneclean Kit BI0101 P. O. Box 2284, La Jolla, California, USA, 92038-2284) gereinigt. Dieses Fragment wurde in den EcoRI-SalI-geschnittenen und phosphatase-behandelten Phagemid-Vektor pTZ19U der Firma Bio-Rad Laboratories (Hercules, California, USA) ligiert und so das Plasmid pTZ19U-cysB erhalten (Fig. 2). Nach der Transformation wurden positive Klone über Restriktionsanalyse identifiziert.

Zur Konstruktion eines konstitutiv aktiven cysB-Allels wurde (in Analogie zu der bei Colyer T. E., Kredich N. M., 1994, Mol. Microbiol. 13: 797-805 beschriebenen Mutation im Wildtyp-cysB-Gen von Salmonella typhimurium) die Mutation des Codons 147 des Wildtyp-cysB-Gens zu einem Methionin-Codon mit dem "Mutagene In Vitro Mutagenesis"-Kit der Firma Bio-Rad Laboratories (Hercules, California, USA) durchgeführt. Dabei wurde das Oligonukleotid CysBMut4 (SEQ. ID. NO. 3) verwendet. Die unterstrichenen Basen zeigen die Abweichung von der Wildtyp-Sequenz an.
5'-TTC GCT ATC GCC ATG GAA GCG CTG CAT-3'(SEQ. ID. NO. 3)

Für Aktivitätstests (siehe Beispiel 2) wurden die beiden cysB-Allele als EcoRI-SalI-Fragmente nach Klenow-Behandlung in den *Ec1*136II-geschnittenen, phosphatase-behandelten Vektor pA-CYC184-LH kloniert.

### Beispiel 2: Bestimmung der CysB-Aktivität in vivo

Zur Messung der CysB-Aktivität wurde ein Reportergen-Ansatz gewählt. Hierfür wurde die Kontroll-Region des cysK-Gens mit dem lacZ-Gen, das für das Enzym β-Galaktosidase kodiert, fusioniert. Durch Integration dieser Fusion in Stämme ohne endogene β-Galaktosidase wird dieses Enzym in Abhängigkeit von der CysB-Aktivität gebildet und liefert somit in Form von β-Galaktosidase-Aktivität ein indirektes Maß der CysB-Aktivität.

Für die Konstruktion der Fusion wurde das von Simons et al. beschriebene System verwendet (Simons R. W. et al., 1987, Gene 53: 85-96). Zunächst wurde der Promotorbereich des cysK-Gens einschließlich der ersten fünfzehn Codons unter Verwendung der Oligonukleotidprimer cysKP1 (SEQ. ID. NO: 4) und cysKP3 (SEQ. ID. NO: 5) und 10 ng chromosomaler DNS von Escherichia coli und Pwo-Polymerase mittels der Polymerase-Ketten-Reaktion amplifiziert.
5'-CCG GAA TTC CCG TTG CCG TTT GTG GCG-3'(SEQ. ID. NO: 4)
5'-CGC GGA TCC GTG TGA CCG ATA GTC AGC-3'(SEQ. ID. NO: 5),

Die Bedingungen entsprachen denjenigen, die in Beispiel 1 beschrieben wurden. Das resultierende 317 Basenpaar-Produkt wurde mit den Restriktionsenzymen EcoRI und BamHI nach Herstellerangaben verdaut und mittels präparativer Gelelektrophorese und der Geneclean-Methode gereinigt. Anschließend wurde das Produkt mit dem ebenfalls EcoRI-BamHI-verdauten und phosphatase-behandelten Vektor pRS552 (hinterlegt gemäß Budapester Vertrag bei der Deutschen Sammlung für Mikroorganismen und Zellkulturen, Braunschweig am 14.9.99 unter der Nummer DSM 13034) ligiert. Mittels Elektroporation wurde der Stamm MC4100 (ATCC 35695) mit dem Ligationsansatz transformiert und positive Klone anhand von Restriktionsanalysen identifiziert. Diese enthalten eine translationale cysK-lacZ-Fusion. Das erhaltene Plasmid wurde der Anleitung von Simons et al. folgend mit dem Bakteriophagen λRS45 (hinterlegt gemäß Budapester Vertrag bei der Deutschen Sammlung für Mikroorganismen und Zellkulturen, Braunschweig am 14.9.99 unter der Nummer DSM 13035) rekombiniert und ein homogenes Lysat des rekombinanten Phagen mit der Bezeichnung λKZL300 hergestellt. Mit diesem Phagen wurde der Δlac-Stamm MC4100 infiziert und durch Kanamycin-Selektion lysogene Klone identifiziert (MC4100::λKZL300), die nun für die Messung der CysB-Aktivität verwendet werden konnten.

Zum Vergleich der Wirkung eines multicopy cysB-Gens kloniert in pACYC184-LH und eines cysB(T149M)-Gens wurde MC4100::λKZL300 mit den entsprechenden Plasmiden pACYC-cysB und pACYC-cysB(T149M) transformiert.
Die Stämme wurden in VB-Minimalmedium (3,5 g/l Na(NH₄)HPO₄; 10 g/l KH₂PO₄; 2 g/l Citrat x H₂O; 0,078 g/l MgCl₂; pH mit NaOH auf 6,5 einstellen, 5 g/l Glukose; 5 mg/l Vitamin B₁) mit verschiedenen Schwefelquellen (je 1 mM Schwefel) kultiviert, wobei 15 mg/l Tetracyclin zugesetzt wurde. Die Bestimmung der β-Galaktosidase-Aktivität erfolgte nach der von Miller beschriebenen Methode (Miller J. H., 1972, Experiments in Molecular Genetics, Cold Spring Harbour, New York, 352-355).

Die Ergebnisse sind in Tabelle 1 dargestellt. Es zeigt sich bei der Kontrolle (MC4100::λKZL300 / pACYC184-LH) ein für ein CysB-Wildtyp typisches Regulationsmuster der CysB-Aktivität in Abhängigkeit von der S-Quelle: Die CysB-Aktivität von Zellen gewachsen mit Thiosulfat gegenüber Sulfat beträgt weniger als 75% und die CysB-Aktivität von Zellen gewachsen mit Cystin gegenüber Sulfat beträgt weniger als 20%. Dieses Muster wird bei Vorhandensein eines Wildtyp-cysB-Gens in multicopy auf einem insgesamt gehobenen Aktivitätsniveau erhalten (erfindungsgemäßes Beispiel MC4100::λKZL300 / pACYC-cysB). Das cysB(T149M)-Allel führt dagegen zu einer konstitutiv hohen Aktiviät unter Verlust des typischen Regulationsmusters.

**Tabelle 1**

| Bestimmung der CysB-Aktivität in Form von β-Galaktosidase-Aktivität von Stämmen mit einer chromosomalen cysK-lacZ-Fusion | | | |
|---|---|---|---|
| Stamm | MC4100:: λKZL300 | MC4100:: λKZL300 | MC4100:: λKZL300 |
| Plasmid | pACYC184-LH | pACYC-cysB | pACYC-cysB(T149M) |
| Genotyp | cysB single copy | cysB multicopy | cysB(T149M) multicopy |
| S-Quelle: | β-Galaktosidase -Aktivität in Miller units | | |
| Cystin | 26 | 341 | 3791 |
| Sulfat | 1906 | 2708 | 3824 |
| Thiosulfat | 726 | 1094 | 3595 |

### Beispiel 3: Konstruktion von erfindungsgemäßen Plasmiden

Erfindungsgemäße Organismen zeichnen sich durch einen deregulierten Cysteinstoffwechsel und beispielsweise durch ein cysB-Gen in mehrfacher Kopienzahl aus. Um solche Organismen herzustellen, wurde das Plasmid (pACYC184-cysEX-GAPDH-ORF306) als Grundkonstrukt gewählt. Dieses Plasmid enthält die Elemente feedback-resistentes cysE-Allel und Efflux-Gen zur Deregulierung des Cysteinstoffwechsels. Es ist in der Patentanmeldung EP 0885962 A1 (Beispiel 2D) eingehend beschrieben. In dieses Konstrukt, verdaut mit dem Restriktionsenzym SnaBI sowie phosphatase-behandelt, wurde zwischen das cysEX-Allel und das Efflux-Gen ein cysB-Fragment inseriert. Letzteres wurde durch Restriktion mit den Enzymen EcoRI und BstXI und durch anschließendes Glätten der DNS-Enden mit Klenow-Enzym aus dem Plasmid pTZ19U-cysB gewonnen (Fig. 2). Das Plasmid trägt die Bezeichnung pHC34.

Durch Transformation des Escherichia coli Stammes W3110 (ATCC 27325; Bachmann B. J., 1996, In: Neidhardt F. C. (ed.) Escherichia coli und Salmonella: cellular and molecular biology, American Society for Microbiology, Washington D.C., Chapter 133) entsteht ein erfindungsgemäßer Organismus. Die Transformation wurde mit Hilfe der Elektroporation durchgeführt. Hierbei wurde eine dichte Zellsuspension in eiskalter 10 %-iger Glycerinlösung mit 0,1 µg Plasmid-DNA versetzt und bei 2500 V, 200 Ohm und 12,5 µF einem elektrischen Puls ausgesetzt. Nach dem Transfer des Ansatzes in steriles LB-Medium (1% Trypton, 0,5% Hefeextrakt, 1% NaCl) und Inkubation bei 30 °C für eine Stunde wurden plasmidtragende Klone auf LB-Agarplatten mit 15 µg/ml Tetracyclin selektiert.

Für einen Vergleich der Wirkung des Wildtyp-cysB-Gens gegenüber dem konstitutiven cysB(T149M)-Allel wurde ein analoges Konstrukt (pHC30) erstellt und ebenfalls in den Stamm W3100 eingebracht. Des weiteren diente der in EP 885962 A1 beschriebene Organismus W3110, transformiert mit dem Plasmid pA-CYC184/cysEX-GAPDH-orf306, als Grundkonstrukt zum Vergleich und gleichzeitig zur Abgrenzung gegen den Stand der Technik.

Da es sich bei W3110 um einen Wildtypstamm handelt, sind alle Cystein-Produktionseffekte auf plasmid-kodierte Gene zurückzuführen.

### Beispiel 4: Cystein-Produktion mit erfindungsgemäßen Mikroorganismen

Zum Nachweis der Cystein-Produktion wurden die in Beispiel 3 beschriebenen Mikroorganismen in Fermentern im Fed-Batch-Modus mit kontinuierlicher Glukose- und Thiosulfat-Fütterung kultiviert. Als Vorrichtung diente ein Biostat M-Gerät der Firma Braun Biotech (Melsungen, D) mit einem maximalen Kulturvolumen von 2 1.
Als Vorkultur wurden 20 ml LB-Medium (10 g/l Trypton, 5 g/l Hefeextrakt, 10 g/l NaCl), das zusätzlich 15 mg/l Tetracyclin enthielt, beimpft und bei 30 °C und 150 rpm in einem Schüttler inkubiert. Nach sieben Stunden wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ x 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na-BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 15 mg/l Tetracyclin supplementiert wurde, überführt. Die weitere Inkubation erfolgte bei 30 °C für 17 Stunden bei 150 rpm.

Mit dieser Vorkultur (optische Dichte bei 600 nm von ca. 3) wurde der Fermenter mit 900 ml Fermentationsmedium (15 g/l Glukose; 10 g/l Trypton; 5 g/l Hefeextrakt; 5 g/l (NH₄)₂SO₄; 1,5 g/l KH₂PO₄; 0,5 g/l NaCl; 0,3 g/l MgSO₄ x 7 H₂O; 0,015 g/l CaCl₂ x 2 H₂O; 0,075 g/l FeSO₄ x 7 H₂O; 1 g/l Na₃Citrat x 2 H₂O und 1 ml/l Spurenelementlösung s.o., 5 mg/l Vitamin B1 und 15 mg/l Tetracyclin, eingestellt auf pH 7,0 mit 25 % Ammoniak) beimpft. Während der Fermentation wurde eine Temperatur von 30 °C eingestellt und der pH-Wert durch Zudosierung von 25 % Ammonik bei einem Wert von 7,0 konstant gehalten. Die Kultur wurde mit entkeimter Druckluft bei 1,5 vol/vol/min begast und mit einer Rührerdrehzahl von 200 rpm gerührt. Nach Absinken der Sauerstoffsättigung auf einen Wert von 50 % wurde die Drehzahl über ein Kontrollgerät bis zu einem Wert von 1200 rpm erhöht, um 50 % Sauerstoffsättigung zu erhalten.

Nach zwei Stunden erfolgte eine Zudosierung einer 30 % Na-Thiosulfat-Lösung mit einer Rate von 3 ml/h. Glukose wurde aus einer 56 % Stammlösung zugefüttert, sobald der Gehalt im Fermenter von anfänglich 15 g/l auf ca. 5-10 g/l abgesunken war. Die Glukose-Fütterung erfolgte mit einer Flußrate von 8-14 ml/h, wobei versucht wurde eine Glukosekonzentration von ca. 5-10 g/l konstant zu halten. Die Glukose-Bestimmung wurde mit dem Glukoseanalysator der Firma YSI (Yellow Springs, Ohio, USA) durchgeführt.
Die Produktion von L-Cystein wurde colorimetrisch mit dem Test von Gaitonde (Gaitonde, M. K. (1967), Biochem. J. 104, 627-633) verfolgt. Dabei ist zu berücksichtigen, daß der Test nicht zwischen L-Cystein und dem in EP 0885962 A1 beschriebenen Kondensationsprodukt von L-Cystein und Pyruvat (2-Methylthiazolidin-2,4-dicarbonsäure) diskriminiert. Schwerlösliches Cystin, das durch Oxidation aus L-Cystein entsteht, wurde nach Lösen in 8 % Salzsäure und anschließende Reduktion mit Dithiothreitol (DTT) in verdünnter Lösung bei pH 8,0 ebenfalls als L-Cystein nachgewiesen.

Tabelle 2 zeigt den Produktionsverlauf einer Fermentation von Organismen mit dem in EP 0885962A1 beschriebenen Grundkonstrukt pACYC184/cysEX-GAPDH-ORF306 im Vergleich mit einem erfindungsgemäßen Plasmid pHC34 und einem entsprechenden Konstrukt mit dem cysB(T149M)-Allel, das für ein konstitutiv aktives CysB-Genprodukt kodiert. Es wird deutlich, daß die erfindungsgemäße Einsatz des Wildtyp-cysB-Gens einen positiven Effekt auf die Produktionsleistung ausübt, während das konstitutive Allel cysB(T149M) negative Auswirkung zeigt.

**Tabelle 2**

| Produktion von L-Cystein mit dem erfindungsgemäßen Konstrukt pHC34 bzw. Kontrollkonstrukten. | | | |
|---|---|---|---|
| Plasmid-konstrukt | pA-CYC184/cysEX-GAPDH-ORF306 | pHC34 | pHC30 |
| Genotyp | cysEX orf306 | cysEX cysB orf306 | cysEX cysB(T149M) orf306 |
| Fermentationszeit | L-Cystein- Ausbeute in g/l | | |
| 24 h | 6,3 | 8,0 + 2,6* | 2,0 |
| 48 h | 10,2 + 7,0 * | 10,0 +12,6* | 3,4 |

| | | | |
|---|---|---|---|
| * Die mit einem Stern angegebenen Werte stehen für L-Cystein, das in oxidierter Form als schwerlösliches Cystin vorliegt. | | | |

## Patentansprüche

1. Mikroorganismenstamm der zur fermentativen Herstellung von L-Cystein oder L-Cystein-Derivaten geeignet ist und einen deregulierten Cysteinstoffwechsel besitzt, wobei diese Deregulation des Cysteinstoffwechsels nicht auf einer geänderten CysB-Aktivität beruht, **dadurch gekennzeichnet, daß** er zusätzlich eine erhöhte CysB-Aktivität besitzt, wobei die CysB-Aktivität ein für ein Wildtyp-CysB typisches Regulationsmuster besitzt.

2. Mikroorganismenstamm der zur fermentativen Herstellung von L-Cystein oder L-Cystein-Derivaten geeignet ist und einen deregulierten Cysteinstoffwechsel besitzt, wobei diese Deregulation des Cysteinstoffwechsels nicht auf einer veränderten CysB-Aktivität beruht, in dem homologe oder heterologe cysB-Gene verstärkt exprimiert werden, die für CysB mit einem für Wildtyp-CysB typischen Regulationsmuster kodieren.

3. Mikroorganismenstamm nach Anspruch 2, **dadurch gekennzeichnet, daß** es sich um einen Escherichia coli Stamm mit dereguliertem Cysteinstoffwechsel handelt, in dem ein Wildtyp-cysB-Gen überexprimiert wird.

4. Mikroorganismenstamm nach Anspruch 3, **dadurch gekennzeichnet, daß** es sich um Escherichia coli Stämme mit dereguliertem Cysteinstoffwechsel handelt, in dem die Kopienzahl des Wildtyp-cysB-Gens von Escherichia coli erhöht ist und dieses Gen überexprimiert wird.

5. Verfahren zur Herstellung eines Mikroorganismus gemäß einem der Ansprüche 1 bis 4, **dadurch gekennzeichnet, daß** in einem Mikroorganismenstamm mit dereguliertem Cysteinstoffwechsel mittels an sich bekannter Methoden die Kopienzahl eines Wildtyp-cysB-Gens oder eines cysB-Gens kodierend für CysB mit einem für Wildtyp-CysB typischen Regulationsmuster erhöht wird, oder daß mittels an sich bekannter Methoden eine verstärkte Expression des Wildtyp-cysB-Gens oder eines cysB-Gens kodierend für ein CysB mit einem Wildtyp-CysB typischen Regulationsmuster bewirkt wird.

6. Verfahren zur Herstellung von L-Cystein, oder L-Cystein-Derivaten **dadurch gekennzeichnet, daß** ein Mikroorganismenstamm gemäß einem der Ansprüche 1 bis 4 in an sich bekannter Art und Weise in der Fermentation eingesetzt wird und das L-Cystein oder L-Cystein-Derivat aus dem Fermentationsansatz abgetrennt wird.

7. Plasmid, **dadurch gekennzeichnet, daß** es genetische Elemente zur Deregulierung des Cysteinstoffwechsels besitzt, wobei diese genetischen Elemente keine Veränderung der CysB-Aktivität bewirken und nicht für eine Topoisomerase I kodieren, sowie ein cysB-Gen kodierend für CysB mit einem für. Wildtyp-CysB typischen Regulationsmuster unter Kontrolle eines Promotors enthält.

8. Verfahren zur Herstellung eines Mikroorganismenstammes gemäß einem der Ansprüche 1 bis 4, **dadurch gekennzeichnet, daß** in einen Mikroorganismenstamm ein Plasmid gemäß Anspruch 7 eingebracht wird.

## Claims

1. Microorganism strain which is suitable for fermentatively producing L-cysteine or L-cysteine derivatives and possesses a deregulated cysteine metabolism, with this deregulation of the cysteine metabolism not being based on a change in CysB activity, **characterized in that** the strain additionally possesses an increased CysB activity, with the CysB activity having a regulatory pattern which is typical of a wild-type CysB.

2. Microorganism strain which is suitable for fermentatively producing L-cysteine or L-cysteine derivatives and possesses a deregulated cysteine metabolism, with this deregulation of the cysteine metabolism not being based on a change in CysB activity, in which strain homologous or heterologous CysB genes, which encode CysB having a regulatory pattern which is typical of wild-type CysB, are expressed to increased extent.

3. Microorganism strain according to Claim 2, **characterized in that** it is an Escherichia coli strain which possesses a deregulated cysteine metabolism and in which a wild-type cysB gene is overexpressed.

4. Microorganism strain according to Claim 3, **characterized in that** it is an Escherichia coli strain which possesses a deregulated cysteine metabolism and in which the copy number of the Escherichia coli wild-type cysB gene is increased and this gene is overexpressed.

5. Process for preparing a microorganism according to one of Claims 1 to 4, **characterized in that**, in a microorganism strain possessing a deregulated cysteine metabolism, the copy number of a wild-type cysB gene, or of a cysB gene which encodes CysB having a regulatory pattern which is typical of wild-type CysB, is increased using methods which are known per se, or **in that** an increased expression of the wild-type cysB gene, or of a cysB gene which encodes a CysB having a regulatory pattern which is typical of wild-type CysB, is brought about using methods which are known per se.

6. Process for producing L-cysteine or L-cysteine derivatives, **characterized in that** a microorganism strain according to one of Claims 1 to 4 is employed in the fermentation in a manner known per se and the L-cysteine or L-cysteine derivative is separated off from the fermentation mixture.

7. Plasmid, **characterized in that** it possesses genetic elements for deregulating cysteine metabolism, with these genetic elements not bringing about any change in CysB activity and not encoding a topoisomerase I, and also contains a cysB gene, which encodes CysB having a regulatory pattern which is typical of wild-type CysB, under the control of a promoter.

8. Process for preparing a microorganism strain according to one of Claims 1 to 4, **characterized in that** a plasmid according to Claim 7 is introduced into a microorganism strain.

## Revendications

1. Souche de microorganismes convenant pour la production par fermentation de L-cystéine ou de dérivés de L-cystéine et qui présente un métabolisme déréglé de la cystéine, ce déréglage du métabolisme de la cystéine ne reposant pas sur une activité CysB modifiée, **caractérisée en ce qu'**elle présente en outre une activité CysB accrue, l'activité CysB présentant un modèle de régulation caractéristique pour un CysB de type sauvage.

2. Souche de microorganismes convenant pour la production par fermentation de L-cystéine ou de dérivés de L-cystéine et qui présente un métabolisme déréglé de la cystéine, ce déréglage du métabolisme de la cystéine ne reposant pas sur une activité CysB modifiée, dans laquelle des gènes homologues ou hétérologues de CysB, qui codent pour CysB avec un modèle de régulation caractéristique pour un CysB de type sauvage, sont exprimés de manière renforcée.

3. Souche de microorganismes selon la revendication 2, **caractérisée en ce qu'**il s'agit d'une souche d'Escherichia coli avec un métabolisme de cystéine déréglé, dans laquelle un gène CysB de type sauvage est surexprimé.

4. Souche de microorganismes selon la revendication 3, **caractérisée en ce qu'**il s'agit de souches d'Escherichia coli avec un métabolisme de cystéine déréglé, dans laquelle le nombre de copies du gène CysB de type sauvage d'Escherichia coli a été augmenté et ce gène est surexprimé.

5. Procédé pour la préparation d'un microorganisme selon l'une quelconque des revendications 1 à 4, **caractérisé en ce que** dans une souche de microorganismes avec un métabolisme de cystéine déréglé, au moyen de méthodes connues en soi, le nombre de copies d'un gène CysB de type sauvage ou d'un gène CysB codant pour le CysB présentant un modèle de régulation caractéristique de CysB de type sauvage est augmenté ou **en ce qu'**au moyen de méthodes connues en soi on provoque une expression renforcée du gène CysB de type sauvage ou d'un gène CysB codant pour un CysB avec un modèle de régulation caractéristique de CysB de type sauvage.

6. Procédé pour la production de L-cystéine ou de dérivés de L-cystéine, **caractérisé en ce qu'**on utilise une souche de microorganismes selon l'une quelconque des revendications 1 à 4 de manière connue en soi dans la fermentation et la L-cystéine ou le dérivé de L-cystéine est séparé de la charge de fermentation.

7. Plasmide **caractérisé en ce qu'**il présente des éléments génétiques pour le déréglage du métabolisme de la cystéine, ces éléments génétiques ne provoquant pas de modification de l'activité de CysB et ne codant pas pour un topoisomérase I et il présente un gène CysB codant pour CysB présentant un modèle de régulation caractéristique de CysB de type sauvage sous contrôle d'un promoteur.

8. Procédé pour la production d'une souche de microorganismes selon l'une quelconque des revendications 1 à 4, **caractérisé en ce qu'**un plasmide selon la revendication 7 est introduit dans une souche de microorganismes.
